# FASCICULE DE BREVET EUROPEEN

(11) **EP 1 066 274 B1**
(45) Date de publication et mention de la délivrance du brevet: **22.09.2004**
(21) Numéro de dépôt: 99911811.0
(22) Date de dépôt: 20.03.1999
(51) Int. Cl.: C07D 301/12

(54) **PROCEDE DE FABRICATION D'UN OXIRANNE**
VERFAHREN ZUR HERSTELLUNG EINES OXIRANS
METHOD FOR MAKING AN OXIRANE

(30) Priorité: 24.03.1998 BE 9800231
(43) Date de publication de la demande: 10.01.2001
(73) Titulaire: SOLVAY (Société Anonyme), 1050 Bruxelles (BE)
(72) Inventeur: CATINAT, Jean-Pierre, B-7131 Waudrez (BE); STREBELLE, Michel, B-1150 Bruxelles (BE)
(74) Mandataire: Vande Gucht, Anne
(86) Numéro de dépôt international: PCT/EP1999/001955
(87) Numéro de publication internationale: WO 1999/048882

(56) Documents cités:
- EP-A- 0 230 949
- EP-A- 0 712 852
- EP-A- 0 757 043
- EP-A- 0 795 537
- CLERICI M G ET AL: "Epoxidation of lower olefins with hydrogen peroxide and titanium silicalite" JOURNAL OF CATALYSIS, vol. 140, no. 1, 1 mars 1993, pages 71-83, XP000562771

## Description

L'invention concerne un procédé de fabrication d'un oxiranne par réaction entre une oléfine et un composé peroxydé en présence d'un catalyseur à base de zéolite. Elle concerne plus particulièrement un procédé de fabrication de 1,2-époxypropane (ou oxyde de propylène) par réaction entre le propylène et le peroxyde d'hydrogène.

Il est connu de fabriquer de l'oxyde de propylène par époxydation de propylène au moyen de peroxyde d'hydrogène et en présence d'un catalyseur de type TS-1, comme décrit par exemple dans la demande de brevet EP 0 230 949. Ce procédé connu présente l'inconvénient de conduire, dans certaines conditions, à des sélectivités et/ou des taux de conversion de peroxyde d'hydrogène trop faibles.

Le document EP 712852 divulgue un procédé d' époxydation d'oléfines sous catalyse de zéolites en présence de solvant Le pH du milieu réactionnel est de 4 à 7,1. Aucun des exemples ne divulgue des valeurs de pH.

Le document EP 757043 divulgue également un procédé d' époxydation d'oléfines sous catalyse de zéolite en présence de solvant On y mentionne l'adjonction de base dans le milieu réactionnel sans toutefois préciser des valeurs de pH.

Le document EP 795537 divulgue un procédé d' époxydation de butadiène sous catalyse de zéolite en présence de solvant. Le pH du milieu réactionnel est de 5 à 7. Des pH de 6,5, 6,2 et 5,9 sont exemplifiés.

Le document Clerici M.G. et al, « Epoxidation of lower olefins with hydrogen peroxide and titanium silicalite » , Journal of Catalysis, vol. 140, n° 1, 1 mars 1993, pages 71-83, concerne un procédé d' époxydation d'oléfines sous catalyse de zéolite en présence d'un solvant. On y mentionne l'adjonction de base dans le milieu réactionnel sans toutefois préciser des valeurs de pH.

L'invention vise à remédier à l'inconvénient précité en fournissant un procédé de fabrication d'un oxiranne qui présente une sélectivité élevée et/ou un taux de conversion élevé.

L'invention concerne dès lors un procédé de fabrication du 1,2-époxypropane par réaction entre le propylène et un composé peroxydé en présence d'un catalyseur à base d'une zéolite et d'un solvant, dans lequel le pH du milieu réactionnel comprenant le propylène, le composé peroxydé, le catalyseur, le 1,2-époxypropane formé et le solvant est de 4,8 à 6,5.

Une des caractéristiques essentielles de l'invention réside dans le pH. En effet, il a été constaté que l'acidité du catalyseur joue un rôle important dans l'obtention d'un bon compromis entre la sélectivité et le taux de conversion du composé peroxydé. En général, une acidité trop élevée conduit à de mauvais résultats. L'acidité du catalyseur est cependant difficile à contrôler au niveau du catalyseur même car des produits qui affectent l'acidité, à savoir des sous-produits formés lors de l'époxydation et des acides entraînés par le recyclage du catalyseur et du solvant et par le propylène non converti, sont facilement adsorbés sur la surface du catalyseur. En outre, ces produits ne sont pas faciles à éliminer lors de la régénération du catalyseur. Il a maintenant été trouvé que le problème d'acidité du catalyseur peut être résolu en maintenant le pH du milieu réactionnel d'époxydation à une valeur d'au moins 4,8, de préférence d'au moins 5. Le pH ne devrait pas dépasser la valeur de 6,5, de préférence 6. Ceci permet en effet d'obtenir un bon compromis entre la sélectivité et le taux de conversion du composé peroxydé. De bons résultats sont obtenus lorsque le pH du milieu réactionnel est maintenu de 4,8 à 6,5, de préférence de 5 à 6.

Dans le procédé selon l'invention le pH du milieu réactionnel peut être contrôlé par addition d'une base. Cette base peut être choisie parmi les bases solubles dans l'eau. Il peut s'agir de bases fortes. On peut citer à titre d'exemples de bases fortes NaOH, KOH ou des hydroxydes d'ammonium quaternaires de formule générale NR₄⁺OH⁻ (R=alkyle). Il peut également s'agir de bases faibles. Les bases faibles peuvent être inorganiques. On peut citer à titre d'exemples de bases faibles inorganiques NH₄OH, Na₂CO₃, NaHCO₃, Na₂HPO₄, K₂CO₃, Li₂CO₃, KHCO₃, LiHCO₃, K₂HPO₄. Les bases faibles peuvent aussi être organiques. Des bases faibles organiques qui peuvent convenir sont les sels de métaux alcalins ou alcalino-terreux d'acides carboxyliques contenant de préférence de 1 à 10 atomes de carbone. Les bases faibles donnent de bons résultats. Les bases faibles organiques sont préférées. L'acétate de sodium convient particulièrement bien.

Les composés peroxydés qui peuvent être utilisés dans le procédé selon l'invention sont les composés peroxydés contenant de l'oxygène actif et capables d'effectuer une époxydation. Le peroxyde d'hydrogène et les composés peroxydés qui peuvent produire du peroxyde d'hydrogène dans les conditions de la réaction d'époxydation conviennent bien. Le peroxyde d'hydrogène est préféré.

Dans le procédé selon l'invention, le composé peroxydé est généralement mis en oeuvre en une quantité d'au moins 1 mol par kg de milieu réactionnel, en particulier d'au moins 1,5 mol par kg de milieu réactionnel. La quantité de composé peroxydé est généralement inférieure à 10 mol par kg de milieu réactionnel; elle est habituellement inférieure ou égale à 5 mol par kg de milieu réactionnel, en particulier inférieure où égale à 3 mol par kg de milieu réactionnel.

Dans le procédé selon l'invention le composé peroxydé est avantageusement mis en oeuvre sous forme d'une solution aqueuse. En général, la solution aqueuse contient au moins 10 % en poids de composé peroxydé, en particulier au moins 20 % en poids. Elle contient le plus souvent au maximum 70 % en poids de composé peroxydé, en particulier 50 % en poids.

Dans le procédé selon l'invention le propylène réagit avec le composé peroxydé en présence du catalyseur et du solvant à une température qui est généralement d'au moins 0 °C, en particulier d'au moins 20 °C. La température est généralement inférieure à 150 °C; elle est habituellement inférieure ou égale à 70 °C, en particulier inférieure ou égale à 40 °C.

Dans le procédé selon l'invention, la réaction entre le propylène et le composé peroxydé peut avoir lieu à pression atmosphérique. Elle peut également se dérouler sous pression. Généralement, cette pression n'excède pas 40 bar. Une pression de 20 bar convient bien en pratique.

Les catalyseurs utilisés dans le procédé selon l'invention contiennent une zéolite, à savoir un solide contenant de la silice qui présente une structure cristalline microporeuse. La zéolite est avantageusement exempte d'aluminium. Elle contient de préférence du titane.

La zéolite utilisable dans le procédé selon l'invention peut avoir une structure cristalline de type ZSM-5, ZSM-11, MCM-41 ou de type zéolite bêta. Les zéolites de type ZSM-5 conviennent bien. Celles présentant une bande d'adsorptiôn infrarouge à environ 950-960 cm⁻¹ sont préférées.

Les zéolites qui conviennent particulièrement bien sont les silicalites au titane. Celles répondant à la formule xTiO₂(1-x)SiO₂ dans laquelle x est de 0,0001 à 0,5, de préférence de 0,001 à 0,05 sont performantes. Des matériaux de ce type, connus sous le nom de TS-1 et présentant une structure cristalline de type ZSM-5, donnent des résultats particulièrement favorables.

Le solvant utilisé dans le procédé selon l'invention présente généralement une miscibilité importante avec l'eau. Des solvants qui donnent de bons résultats sont les dérivés organiques aliphatiques contenant de 1 à 4 atomes de carbone. On peut citer à titre d'exemple le méthanol.

Le rapport molaire entre la quantité de propylène engagée et la quantité de composé peroxydé est généralement supérieur ou égal à 1, en particulier supérieur ou égal à 1,5. Ce rapport molaire est le plus souvent inférieur ou égal à 20, en particulier inférieur ou égal à 10.

Lors d'essais en continu, le rapport molaire entre la quantité de propylène engagée et la quantité de solvant est généralement supérieur ou égal à 0,1, de préférence supérieur ou égal à 0,5. Ce rapport molaire est le plus souvent inférieur ou égal à 50, de préférence inférieur ou égal à 10.

Le procédé selon l'invention peut être réalisé en continu. En variante, il peut être réalisé en discontinu.

### Exemples

De l'oxyde de propylène a été fabriqué par réaction entre le propylène et le peroxyde d'hydrogène en présence d'un catalyseur TS-1 et en présence de méthanol. Dans l'exemple 1 donné à titre de comparaison, le pH du milieu réactionnel est maintenu à une valeur inférieure à 4,8. Dans les exemples 2 à 4 conformes à l'invention, le pH du milieu réactionnel est maintenu à un pH de 4,8 à 6,5 par addition d'acétate de sodium.

Les résultats sont rassemblés dans le tableau 1 ci-dessous. Les essais ont été réalisés en batch à une température de 35 °C, avec un débit en propylène de 10 mol/h pour 0,6 mol de peroxyde d'hydrogène ajouté sous forme d'une solution aqueuse contenant 35 % en poids de peroxyde d'hydrogène. La quantité de méthanol mise en oeuvre était de 14,4 mol/mol H₂O₂ (360 ml). Le catalyseur a été mis en oeuvre en une quantité de 6,8 g.

Dans les exemples qui suivent la vitesse de conversion du peroxyde d'hydrogène est exprimée par la constante k de vitesse d'ordre 1 répondant à la relation : vitesse = k x (concentration en H₂O₂). La sélectivité est donnée par le rapport entre la quantité d'oxiranne obtenue divisée par la somme de tous les produits formés.

**Tableau 1**

| Exemple | pH | sélectivité | k (min⁻¹) |
|---|---|---|---|
| 1 | 4,0 | 84,0 | 59 |
| 2 | 5,5 | 90,7 | 26 |
| 3 | 6,0 | 97,4 | 15 |
| 4 | 6,3 | 98,1 | 1,6 |

## Revendications

1. Procédé de fabrication de 1,2-époxypropane par réaction entre le propylène et un composé peroxydé en présence d'un catalyseur à base d'une zéolite et en présence d'un solvant, dans lequel le pH du milieu réactionnel comprenant le propylène, le composé peroxydé, le catalyseur, le 1,2-époxypropane formé et le solvant est de 4,8 à 6,5.

2. Procédé selon la revendication 1, dans lequel le pH du milieu réactionnel est de 5 à 6.

3. Procédé selon la revendication 1 ou 2, dans lequel le composé peroxydé est mis en oeuvre en une quantité de 1 à 10 mol, de préférence de 1,5 à 5 mol, par kg de milieu réactionnel.

4. Procédé selon l'une quelconque des revendications précédentes, dans lequel le composé peroxydé est mis en oeuvre sous forme d'une solution aqueuse contenant de 10 à 70 % de composé peroxydé, de préférence de 20 à 50 %.

5. Procédé selon l'une quelconque des revendications précédentes, dans lequel la réaction est réalisée à une température de 0 à 150 °C, généralement de 0 à 70 °C, de préférence de 20 à 40 °C.

6. Procédé selon l'une quelconque des revendications précédentes, dans lequel le pH du milieu réactionnel est maintenu dans la zone de 4,8 à 6,5 par l'addition d'une base.

7. Procédé selon la revendication 6, dans lequel la base est choisie parmi les bases faibles.

8. Procédé selon la revendication 7, dans lequel la base est l'acétate de sodium.

9. Procédé selon l'une quelconque des revendications précédentes, dans lequel la zéolite est du silicalite au titane, de préférence de type TS-1 présentant une structure cristalline de type ZSM-5.

10. Procédé selon l'une quelconque des revendications précédentes, dans lequel le composé peroxydé est le peroxyde d'hydrogène et le solvant est le méthanol.

## Claims

1. Process for manufacturing 1,2-epoxypropane by reaction between propylene and a peroxide compound in the presence of a zeolite-based catalyst and in the presence of a solvent, in which the pH of the reaction medium comprising propylene, the peroxide compound, the catalyst, the 1,2-epoxypropane formed and the solvent is from 4.8 to 6.5.

2. Process according to Claim 1, in which the pH of the reaction medium is from 5 to 6.

3. Process according to Claim 1 or 2, in which the peroxide compound is used in an amount of from 1 to 10 mol, preferably from 1.5 to 5 mol, per kg of reaction medium.

4. Process according to any one of the preceding claims, in which the peroxide compound is used in the form of an aqueous solution containing from 10 to 70% of peroxide compound, preferably from 20 to 50%.

5. Process according to any one of the preceding claims, in which the reaction is carried out at a temperature of from 0 to 150°C, generally from 0 to 70°C, preferably from 20 to 40°C.

6. Process according to any one of the preceding claims, in which the pH of the reaction medium is maintained in the zone from 4.8 to 6.5 by the addition of a base.

7. Process according to Claim 6, in which the base is chosen from weak bases.

8. Process according to Claim 7, in which the base is sodium acetate.

9. Process according to any one of the preceding claims, in which the zeolite is titanium silicalite, preferably of TS-1 type with a crystalline structure of ZSM-5 type.

10. Process according to any one of the preceding claims, in which the peroxide compound is hydrogen peroxide and the solvent is methanol.

## Patentansprüche

1. Verfahren zur Herstellung von 1,2-Epoxypropan durch Reaktion zwischen Propylen und einer Peroxidverbindung in Gegenwart eines Katalysators auf Zeolithbasis und in Gegenwart eines Lösungsmittels, worin der pH-Wert des das Propylen, die Peroxidverbindung, den Katalysator, das gebildete 1,2-Epoxypropan und das Lösungsmittel umfassenden Reaktionsmilieus von 4,8 bis 6,5 beträgt.

2. Verfahren nach Anspruch 1, worin der pH-Wert des Reaktionsmilieus von 5 bis 6 beträgt.

3. Verfahren nach Anspruch 1 oder 2, worin die Peroxidverbindung in einer Menge von 1 bis 10 Mol, vorzugsweise von 1,5 bis 5 Mol je kg des Reaktionsmilieus eingesetzt wird.

4. Verfahren nach einem der vorstehenden Ansprüche, worin die Peroxidverbindung in Form einer wäßrigen Lösung mit einem Gehalt an 10 bis 70% Peroxidverbindung, vorzugsweise 20 bis 50% Peroxidverbindung eingesetzt wird.

5. Verfahren nach einem der vorstehenden Ansprüche, worin die Reaktion bei einer Temperatur von 0 bis 150°C, im allgemeinen von 0 bis 70°C, vorzugsweise von 20 bis 40°C ausgeführt wird.

6. Verfahren nach einem der vorstehenden Ansprüche, worin der pH-Wert des Reaktionsmilieus durch Zugabe einer Base im Bereich von 4,8 bis 6,5 gehalten wird.

7. Verfahren nach Anspruch 6, worin die Base unter den schwachen Basen ausgewählt wird.

8. Verfahren nach Anspruch 7, worin die Base das Natriumacetat ist.

9. Verfahren nach einem der vorstehenden Ansprüche, worin der Zeolith ein Titansilicalit ist, vorzugsweise vom Typ TS-1, der eine kristalline Struktur vom Typ ZSM-5 aufweist.

10. Verfahren nach einem der vorstehenden Ansprüche, worin die Peroxidverbindung das Wasserstoffperoxid ist und das Lösungsmittel Methanol ist.
